# FASCICULE DE BREVET EUROPEEN

(11) **EP 4 457 034 B1**
(45) Date de publication et mention de la délivrance du brevet: **01.04.2026**
(21) Numéro de dépôt: 22847586.9
(22) Date de dépôt: 20.12.2022
(51) Int. Cl.: B05B 12/08

(54) **PROCEDE ET DISPOSITIF D'ANALYSE D'UN DISPOSITIF DE PULVERISATION DE PRODUIT FLUIDE PHARMACEUTIQUE**
VERFAHREN UND VORRICHTUNG ZUR ANALYSE EINER VORRICHTUNG ZUM SPRÜHEN EINES PHARMAZEUTISCHEN FLÜSSIGPRODUKTS
METHOD AND DEVICE FOR ANALYSING A DEVICE FOR SPRAYING A PHARMACEUTICAL FLUID PRODUCT

(30) Priorité: 30.12.2021 FR 2114687
(43) Date de publication de la demande: 06.11.2024
(73) Titulaire: APTAR France SAS, 27110 Le Neubourg (FR)
(72) Inventeur: GRANJON, Guillaume, 27400 La Vacherie (FR)
(74) Mandataire: CAPRI
(86) Numéro de dépôt international: PCT/FR2022/052429
(87) Numéro de publication internationale: WO 2023/126596

(56) Documents cités:
- US-A1- 2016 216 108
- US-A1- 2019 376 106

## Description

La présente invention concerne un dispositif et un procédé d'analyse de spray généré par un dispositif de pulvérisation de produit fluide pharmaceutique.

Les dispositifs de pulvérisation de produit fluide pharmaceutique sont bien connus. Ils comportent généralement une tête de pulvérisation pourvue d'un orifice de pulvérisation, assemblée sur un réservoir contenant le produit fluide à distribuer. En particulier dans des applications de pulvérisation nasale, l'efficacité thérapeutique du produit fluide pulvérisé peut dépendre des propriétés du spray généré lors de l'actionnement du dispositif. De manière connue, à la fin de la chaîne de montage, c'est-à-dire lorsque le dispositif de pulvérisation est assemblé, et juste avant d'être expédié chez le fabriquant du produit fluide pharmaceutique pour y être assemblé sur un réservoir correspondant, un certain nombre d'échantillons de dispositifs assemblés sont testés en laboratoire pour vérifier si les propriétés du spray correspondent au cahier des charges prédéfini.

Un inconvénient de ce système est qu'il concerne des dispositifs assemblés, et donc destructeur de ces dispositifs qui ne pourront plus, après avoir été testés, être livrés au client.

De plus, ce système impose une vérification humaine des dispositifs testés, et n'est donc pas totalement automatisable.

Pour surmonter cet inconvénient, le document WO2018130791 propose la visualisation par strioscopie d'un flux d'air comprimé chaud ou froid envoyé à travers une tête de pulvérisation. Cette méthode permet d'évaluer l'angle du spray mais pas sa géométrie ni sa symétrie. Ceci a de plus l'inconvénient d'avoir à prévoir un banc strioscopique, relativement complexe et coûteux, et difficilement adaptable dans une chaine de montage d'un dispositif de pulvérisation de produit fluide, et implique donc soit des tests aléatoires sur une partie seulement des dispositifs fabriqués, soit un ralentissement de la chaine de montage, généralement peu souhaitable.

Les documents EP3047912, JPH0599802 et JPS54127347 décrivent d'autres dispositifs de l'état de la technique.

La présente invention a pour but de surmonter les inconvénients susmentionnés.

En particulier, la présente invention a pour but de fournir un dispositif et un procédé d'analyse d'un dispositif de pulvérisation de produit fluide pharmaceutique qui soit non destructeur des dispositifs testés.

La présente invention a également pour but de fournir un dispositif et un procédé d'analyse qui soit automatisé en grande partie.

La présente invention a aussi pour but de fournir un dispositif et un procédé d'analyse qui permette de tester 100% des dispositifs de pulvérisation, sans ralentir la chaine de montage de manière substantielle.

La présente invention a également pour but de fournir un dispositif et un procédé d'analyse qui soit simple et/ou peu coûteux à fabriquer, à assembler et à utiliser.

La présente a donc pour objet un procédé d'analyse d'un dispositif de pulvérisation de produit fluide pharmaceutique comprenant les étapes suivantes :
- fournir une tête de pulvérisation d'un dispositif de pulvérisation de produit fluide pharmaceutique, ladite tête de pulvérisation comportant un orifice de pulvérisation,
- fournir une surface de réception comportant une pluralité de zones de contact discrètes séparées par des vides, lesdites zones de contact étant électriquement conductrices,
- faire passer un flux de gaz ionisé à travers ledit orifice de pulvérisation de ladite tête de pulvérisation, ledit flux de gaz ionisé étant chargé en électrons,
- envoyer ledit flux de gaz ionisé sur ladite surface de réception,
- visualiser la zone d'impact dudit flux de gaz ionisé sur ladite surface de réception, et
- analyser ladite visualisation de ladite zone d'impact pour déterminer si ladite zone d'impact est conforme ou non conforme à des spécifications prédéterminées.

Avantageusement, ledit flux de gaz ionisé est un flux de gaz comprimé.

Avantageusement, ledit flux de gaz ionisé est un flux d'air pulsé ionisé.

Avantageusement, ladite étape d'analyser comprend de déterminer la géométrie, notamment la symétrie, de la zone d'impact dudit flux de gaz comprimé sur ladite surface de réception.

Avantageusement, lesdites spécifications prédéterminées comprennent une étendue planaire prédéterminée de la zone d'impact dudit flux de gaz ionisé sur ladite surface de réception, de sorte que les têtes de pulvérisation pour lesquelles ladite étendue planaire est similaire à ladite étendue planaire prédéterminée sont classées conformes, et les têtes de pulvérisation pour lesquelles ladite étendue planaire est différente de ladite étendue planaire prédéterminée sont classés non conformes.

Avantageusement, un cycle d'utilisation comprend les étapes suivantes:
- relier ladite surface de réception à la terre pour en supprimer toutes charges électriques,
- générer ledit flux de gaz ionisé et l'envoyer à travers ladite tête de pulvérisation sur ladite surface de réception,
- détecter chaque zone de contact qui se charge d'ions négatifs au contact dudit flux de gaz ionisé, et
- visualiser ladite zone d'impact au moyen d'une interface homme-machine.

La présente a également pour objet un dispositif d'analyse d'un dispositif de pulvérisation de produit fluide pharmaceutique comprenant :
- une tête de pulvérisation d'un dispositif de pulvérisation de produit fluide pharmaceutique, ladite tête de pulvérisation comportant un orifice de pulvérisation,
- une surface de réception comportant une pluralité de zones de contact discrètes séparées par des vides, lesdites zones de contact étant électriquement conductrices,
- des moyens de mise à la terre pour supprimer toutes charges électriques de ladite surface de réception avant chaque analyse,
- des moyens de génération d'un flux de gaz ionisé pour faire passer un flux de gaz ionisé à travers ledit orifice de pulvérisation de ladite tête de pulvérisation et l'envoyer sur ladite surface de réception, ledit flux de gaz ionisé étant chargé en électrons,
- des moyens de traitement pour visualiser la zone d'impact dudit flux de gaz ionisé sur ladite surface de réception, et
- des moyens d'analyse pour analyser ladite visualisation de ladite zone d'impact pour déterminer si ladite zone d'impact est conforme ou non conforme à des spécifications prédéterminées.

Avantageusement, ledit flux de gaz ionisé est un flux d'air pulsé ionisé.

Avantageusement, lesdits moyens de traitement comportent un amplificateur et des moyens de mesure de différence de potentiel pour chaque zone de contact.

Avantageusement, chaque zone de contact de ladite surface de réception est reliée auxdits moyens de traitement par un câble à faible impédance respectif.

Avantageusement, ladite surface de réception est formée par les extrémités d'une pluralité de pointes disposées en réseau, lesdites extrémités formant lesdites zones de contact.

Avantageusement, lesdites pointes sont solidaires d'une base reliée à la terre.

Avantageusement, lesdites pointes sont équidistantes et proches les unes des autres, formant ainsi un réseau régulier et dense de zones de contact sur ladite surface de réception.

Avantageusement, lesdits moyens de génération du flux de gaz ionisé sont adaptés à générer des impulsions de durée réglable, notamment de 50 à 300 ms.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique d'un dispositif pour analyser un dispositif de pulvérisation, selon un mode de réalisation avantageux, avant utilisation,
la figure 2 est une vue schématique similaire à celle de la figure 1, en cours d'utilisation,
la figure 3 montre une visualisation d'une zone d'impact conforme, et
la figure 4 montre une visualisation d'une zone d'impact non-conforme.

Un objectif de l'invention est d'améliorer la qualité du contrôle des dispositifs de pulvérisation. Pour cela, l'invention prévoit d'analyser 100% des dispositifs, sans ralentissement substantiel de la chaine de montage.

De manière classique, chaque dispositif de pulvérisation comprend une tête de pulvérisation 1 pourvue d'un orifice de pulvérisation 2. Généralement, un profil de pulvérisation (non représenté) est prévu en amont dudit orifice de pulvérisation 2 pour générer une forme de spray conique en sortie de l'orifice.

La présente invention prévoit de faire passer un flux de gaz ionisé F1, de préférence comprimé, à travers chaque tête de pulvérisation 1, et de diriger ce flux F1, sortant de l'orifice de pulvérisation 2 avec la forme d'un spray conique, vers une surface de réception 10. Avantageusement, le flux de gaz ionisé F1 est un flux d'air ionisé, mais il est entendu que selon l'invention, n'importe quel gaz approprié autre que de l'air pourrait être utilisé.

Le flux de gaz ionisé F1 est chargé en électrons, et au moment où ce flux F1 est expulsé à travers l'orifice de pulvérisation 2, la surface de réception 10 est dépourvue de charges électriques.

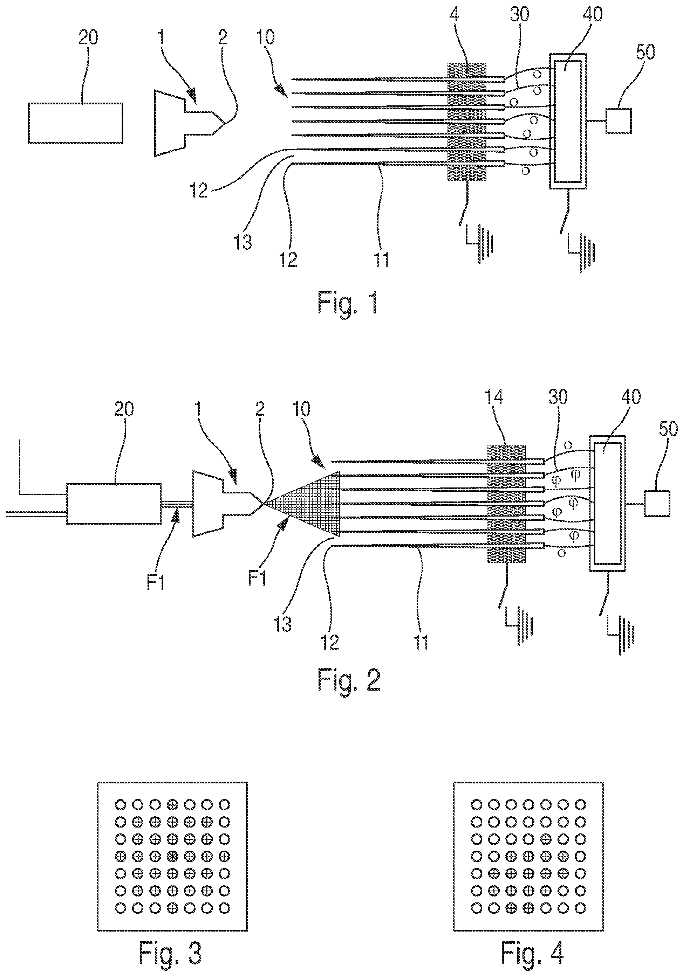

Les figures 1 et 2 montrent un dispositif de test selon un mode de réalisation avantageux.

Dans cet exemple, une tête de pulvérisation 1 est disposée en face d'une surface de réception 10. Des moyens de génération 20 d'un flux de gaz ionisé F1 sont prévus pour faire passer un flux de gaz ionisé F1 chargé en électrons à travers la tête de pulvérisation 1.

La surface de réception 10 forme un plan qui comporte une pluralité de zones de contact 12 séparées les unes des autres par une pluralité de vides 13. Chaque zone de contact 12 est électriquement conductrice.

Dans l'exemple représenté, la surface de réception 10 est formée par les extrémités d'une pluralité de pointes 11 disposées en réseau. Ces extrémités forment alors les zones de contact 12. Chaque pointe 11 forme ainsi une anode fonctionnant tel un paratonnerre. Ces pointes 11 peuvent être solidaires d'une base 14, de préférence isolante, et qui peut avant chaque analyse être reliée à la terre pour éliminer toutes charges électriques de la surface de réception 10.

Avantageusement, les pointes 11 sont équidistantes, et proches les unes des autres, formant ainsi un réseau régulier et dense de zones de contact 12 sur la surface de réception 10. Plus il y a de pointes 11 et plus les zones de contact 12 sont petites, plus la définition de la zone d'impact du flux de gaz ionisé F1 sur la surface de réception 10 est précise et la forme de cette zone d'impact bien retranscrite.

La forme particulière de la surface de réception 10, avec une pluralité de zones de contact discrètes 12 séparées par des vides 13 permet un contact local du flux de gaz ionisé F1 sur les zones de contact 12, sans dispersions et sans perturbations du flux, ce qui rend visible la zone d'impact avec une grande fiabilité.

Pour réaliser les évaluations de conformité, on prévoit des moyens de traitement 40 pour visualiser la zone d'impact et des moyens d'analyse 50 pour analyser les visualisations générées par les moyens de traitement 40 et ainsi déterminer si la zone d'impact du flux de gaz ionisé F1 issu de ladite tête de pulvérisation 1 sur la surface de réception 10 est conforme ou non conforme à des spécifications prédéterminées.

Les moyens de traitement 40 peuvent comporter un amplificateur pour amplifier les signaux électriques reçu de la surface de réception 10. Avantageusement, chaque zone de contact 12 électriquement conductrice est reliée auxdits moyens de traitement 40 par un câble à faible impédance respectif 30. Les moyens de traitement 40 peuvent ainsi calculer des différences de potentiels pour chaque zone de contact 12 qui s'est chargée en ions négatifs au contact du flux de gaz ionisé F1 et ainsi créer une matrice formant une visualisation de la zone d'impact.

La durée de l'impulsion de gaz ionisé F1 est avantageusement réglable, notamment de 50 à 300 ms.

Avantageusement, on réalise plusieurs cycles successifs sur une même tête de pulvérisation, par exemple cinq cycles. La constance ou répétabilité des résultats permet également d'évaluer la conformité de ladite tête de pulvérisation.

Les spécifications prédéterminées peuvent comprendre une étendue planaire prédéterminée de ladite zone d'impact sur ladite surface de réception 10, de sorte que les têtes de pulvérisation 1 pour lesquelles ladite étendue planaire est similaire à ladite étendue planaire prédéterminée sont classées conformes, et les têtes de pulvérisation 1 pour lesquelles ladite étendue planaire est différente de ladite étendue planaire prédéterminée sont classés non conformes. La géométrie, et notamment la symétrie, de la zone d'impact peut aussi être utilisée dans l'évaluation de conformité. D'autres paramètres peuvent aussi être envisagés.

Les moyens d'analyse 50 peuvent comprendre des moyens de mesure de la géométrie de la zone d'impact du flux de gaz ionisé F1 sur la surface de réception 10. Par exemple, on détermine le barycentre de la zone d'impact, et on mesure les distances maximale et minimale de ce barycentre du bord de la zone d'impact. La comparaison de ces distances avec des valeurs prédéterminées permet alors d'évaluer la conformité du dispositif testé. Ainsi, l'évaluation de conformité tient compte non seulement de la surface de la zone d'impact, mais aussi de sa géométrie, en particulier sa symétrie. Ceci permet d'établir qu'un spray sortant d'une tête de pulvérisation conforme aura une forme conique acceptable, tant d'un point de vue de l'angle du spray que de sa symétrie.

Eventuellement, des moyens de traitement d'image peuvent être utilisés pour réaliser ce type d'analyse.

Les figures 3 et 4 illustrent chacune une représentation schématique obtenue avec le procédé et le dispositif de l'invention, sur lesquelles il est possible d'évaluer l'étendue planaire et la géométrie, notamment la symétrie, de la zone d'impact. La figure 3 montre une visualisation de la zone d'impact pour un dispositif conforme et la figure 4 montre une telle visualisation pour un dispositif non-conforme.

La présente invention présente de nombreux avantages, et notamment :
- elle permet un contrôle automatisé de conformité sur divers types de dispositif de pulvérisation ;
- elle permet une analyse non destructive desdits dispositifs de pulvérisation ;
- elle permet d'analyser 100% des dispositifs de pulvérisation assemblés sur une chaine de montage, sans ralentissement substantiel de celle-ci ;
- elle permet de réaliser plusieurs tests successifs sur un même dispositif pour évaluer la répétabilité des résultats ;
- elle utilise un montage compact et facilement adaptable ;
- elle utilise des composants simples et standards, donc généralement peu coûteux ;
- elle permet un traitement d'image robuste, qui peut être réalisé en temps réel ;
- elle assure une bonne répétabilité et une bonne discrimination des dispositifs conformes et non-conformes.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Procédé d'analyse d'un dispositif de pulvérisation de produit fluide pharmaceutique, **caractérisé en ce qu'**il comprend les étapes suivantes :
- fournir une tête de pulvérisation (1) d'un dispositif de pulvérisation de produit fluide pharmaceutique, ladite tête de pulvérisation (1) comportant un orifice de pulvérisation (2),
- fournir une surface de réception (10) comportant une pluralité de zones de contact discrètes (12) séparées par des vides (13), lesdites zones de contact (12) étant électriquement conductrices,
- faire passer un flux de gaz ionisé (F1) à travers ledit orifice de pulvérisation (2) de ladite tête de pulvérisation (1), ledit flux de gaz ionisé (F1) étant chargé en électrons,
- envoyer ledit flux de gaz ionisé (F1) sur ladite surface de réception (10),
- visualiser la zone d'impact dudit flux de gaz ionisé (F1) sur ladite surface de réception (10), et
- analyser ladite visualisation de ladite zone d'impact pour déterminer si ladite zone d'impact est conforme ou non conforme à des spécifications prédéterminées.

2. Procédé selon la revendication 1, dans lequel ledit flux de gaz ionisé (F1) est un flux de gaz comprimé.

3. Procédé selon la revendication 1 ou 2, dans lequel ledit flux de gaz ionisé (F1) est un flux d'air pulsé ionisé.

4. Procédé selon l'une quelconque des revendications précédentes, dans lequel ladite étape d'analyser comprend de déterminer la géométrie, notamment la symétrie, de la zone d'impact dudit flux de gaz comprimé (F1) sur ladite surface de réception (10).

5. Procédé selon l'une quelconque des revendications précédentes, dans lequel lesdites spécifications prédéterminées comprennent une étendue planaire prédéterminée de la zone d'impact dudit flux de gaz ionisé (F1) sur ladite surface de réception (10), de sorte que les têtes de pulvérisation (1) pour lesquelles ladite étendue planaire est similaire à ladite étendue planaire prédéterminée sont classées conformes, et les têtes de pulvérisation (1) pour lesquelles ladite étendue planaire est différente de ladite étendue planaire prédéterminée sont classés non conformes.

6. Procédé selon l'une quelconque des revendications précédentes, dans lequel un cycle d'utilisation comprend les étapes suivantes :
- relier ladite surface de réception (10) à la terre pour en supprimer toutes charges électriques,
- générer ledit flux de gaz ionisé (F1) et l'envoyer à travers ladite tête de pulvérisation (1) sur ladite surface de réception (10),
- détecter chaque zone de contact (12) qui se charge d'ions négatifs au contact dudit flux de gaz ionisé (F1), et
- visualiser ladite zone d'impact au moyen d'une interface homme-machine.

7. Dispositif d'analyse d'un dispositif de pulvérisation de produit fluide pharmaceutique, **caractérisé en ce qu'**il comprend :
- une tête de pulvérisation (1) d'un dispositif de pulvérisation de produit fluide pharmaceutique, ladite tête de pulvérisation (1) comportant un orifice de pulvérisation (2),
- une surface de réception (10) comportant une pluralité de zones de contact discrètes (12) séparées par des vides (13), lesdites zones de contact (12) étant électriquement conductrices,
- des moyens de mise à la terre pour supprimer toutes charges électriques de ladite surface de réception (10) avant chaque analyse,
- des moyens de génération (20) d'un flux de gaz ionisé (F1) pour faire passer un flux de gaz ionisé (F1) à travers ledit orifice de pulvérisation (2) de ladite tête de pulvérisation (1) et l'envoyer sur ladite surface de réception (10), ledit flux de gaz ionisé (F1) étant chargé en électrons,
- des moyens de traitement (40) pour visualiser la zone d'impact dudit flux de gaz ionisé (F1) sur ladite surface de réception (10), et
- des moyens d'analyse (50) pour analyser ladite visualisation de ladite zone d'impact pour déterminer si ladite zone d'impact est conforme ou non conforme à des spécifications prédéterminées.

8. Dispositif selon la revendication 7, dans lequel ledit flux de gaz ionisé (F1) est un flux d'air pulsé ionisé.

9. Dispositif selon la revendication 7 ou 8, dans lequel lesdits moyens de traitement (40) comportent un amplificateur et des moyens de mesure de différence de potentiel pour chaque zone de contact (12).

10. Dispositif selon l'une quelconque des revendications 7 à 9, dans lequel chaque zone de contact (12) de ladite surface de réception (10) est reliée auxdits moyens de traitement (40) par un câble à faible impédance (30) respectif.

11. Dispositif selon l'une quelconque des revendications 7 à 10, dans lequel ladite surface de réception (10) est formée par les extrémités d'une pluralité de pointes (11) disposées en réseau, lesdites extrémités formant lesdites zones de contact (12).

12. Dispositif selon la revendication 11, dans lequel lesdites pointes (11) sont solidaires d'une base (14) reliée à la terre.

13. Dispositif selon la revendication 11 ou 12, dans lequel lesdites pointes (11) sont équidistantes et proches les unes des autres, formant ainsi un réseau régulier et dense de zones de contact (12) sur ladite surface de réception (10).

14. Dispositif selon l'une quelconque des revendications 7 à 13, dans lequel lesdits moyens de génération (20) du flux de gaz ionisé (F1) sont adaptées à générer des impulsions de durée réglable, notamment de 50 à 300 ms.

## Patentansprüche

1. Analyseverfahren einer Vorrichtung zum Versprühen eines pharmazeutischen Flüssigprodukts, **dadurch gekennzeichnet, dass** es folgende Schritte umfasst:
- Bereitstellen eines Sprühkopfs (1) einer Vorrichtung zum Versprühen eines pharmazeutischen Flüssigprodukts, wobei der Sprühkopf (1) eine Sprühöffnung (2) umfasst,
- Bereitstellen einer Aufnahmefläche (10), die eine Vielzahl von diskreten Kontaktbereichen (12) umfasst, die durch Zwischenräume (13) getrennt sind, wobei die Kontaktbereiche (12) elektrisch leitfähig sind,
- Verbreiten eines ionisierten Gasstroms (F1) durch die Sprühöffnung (2) des Sprühkopfs (1) hindurch, wobei der ionisierte Gasstrom (F1) mit Elektronen aufgeladen ist,
- Weiterleiten des ionisierten Gasstroms (F1) auf die Aufnahmefläche (10),
- Visualisieren des Auftreffbereichs des ionisierten Gasstroms (F1) auf der Aufnahmefläche (10), und
- Analysieren der Visualisierung des Auftreffbereichs, um zu bestimmen, ob der Auftreffbereich mit vorbestimmten Spezifikationen konform oder nicht konform ist.

2. Verfahren nach Anspruch 1, wobei der ionisierte Gasstrom (F1) ein Druckgasstrom ist.

3. Verfahren nach Anspruch 1 oder 2, wobei der ionisierte Gasstrom (F1) ein impulsartiger ionisierter Luftstrom ist.

4. Verfahren nach einem der vorhergehenden Ansprüche, wobei der Schritt des Analysierens das Bestimmen der Geometrie, insbesondere der Symmetrie, des Auftreffbereichs des Druckgasstroms (F1) auf der Aufnahmefläche (10) umfasst.

5. Verfahren nach einem der vorhergehenden Ansprüche, wobei die vorbestimmten Spezifikationen eine vorbestimmte planare Erstreckung des Auftreffbereichs des ionisierten Gasstroms (F1) auf der Aufnahmefläche (10) umfassen, so dass die Sprühköpfe (1), für welche die planare Erstreckung ähnlich wie die vorbestimmte planare Erstreckung ist, als konform eingestuft werden, und die Sprühköpfe (1), für welche die planare Erstreckung anders als die vorbestimmte planare Erstreckung ist, als nicht konform eingestuft werden.

6. Verfahren nach einem der vorhergehenden Ansprüche, wobei ein Verwendungszyklus folgende Schritte umfasst:
- Legen der Aufnahmefläche (10) an Masse, um alle elektrischen Ladungen darin zu unterdrücken,
- Generieren des ionisierten Gasstroms (F1) und Weiterleiten desselben durch den Sprühkopf (1) hindurch auf die Aufnahmefläche (10),
- Detektieren jedes Kontaktbereichs (12), der sich beim Kontakt mit dem ionisierten Gasstrom (F1) mit negativen Ionen auflädt, und
- Visualisieren des Auftreffbereichs mittels einer Mensch-Maschine-Schnittstelle.

7. Analysevorrichtung einer Vorrichtung zum Versprühen eines pharmazeutischen Flüssigprodukts, **dadurch gekennzeichnet, dass** sie umfasst:
- einen Sprühkopf (1) einer Vorrichtung zum Versprühen eines pharmazeutischen Flüssigprodukts, wobei der Sprühkopf (1) eine Sprühöffnung (2) umfasst,
- eine Aufnahmefläche (10), die eine Vielzahl von diskreten Kontaktbereichen (12) umfasst, die durch Zwischenräume (13) getrennt sind, wobei die Kontaktbereiche (12) elektrisch leitfähig sind,
- Erdungsmittel zum Unterdrücken eventueller elektrischer Ladungen von der Aufnahmefläche (10) vor jeder Analyse,
- Mittel (20) zum Generieren eines ionisierten Gasstroms (F1), um einen ionisierten Gasstrom (F1) durch die Sprühöffnung (2) des Sprühkopfs (1) hindurch zu verbreiten und auf die Aufnahmefläche (10) weiterzuleiten, wobei der ionisierte Gasstrom (F1) mit Elektronen aufgeladen ist,
- Bearbeitungsmittel (40) zum Visualisieren des Auftreffbereichs des ionisierten Gasstroms (F1) auf der Aufnahmefläche (10), und
- Analysemittel (50) zum Analysieren der Visualisierung des Auftreffbereichs, um zu bestimmen, ob der Auftreffbereich mit den vorbestimmten Spezifikationen konform oder nicht konform ist.

8. Vorrichtung nach Anspruch 7, wobei der ionisierte Gasstrom (F1) ein impulsförmiger ionisierter Luftstrom ist.

9. Vorrichtung nach Anspruch 7 oder 8, wobei die Bearbeitungsmittel (40) einen Verstärker und Mittel zum Messen der Potentialdifferenz für jeden Kontaktbereich (12) umfassen.

10. Vorrichtung nach einem der Ansprüche 7 bis 9, wobei jeder Kontaktbereich (12) der Aufnahmefläche (10) mit den Bearbeitungsmitteln (40) über ein jeweiliges niederohmiges Kabel (30) verbunden ist.

11. Vorrichtung nach einem der Ansprüche 7 bis 10, wobei die Aufnahmefläche (10) durch die Enden einer Vielzahl von Spitzen (11) gebildet ist, die gitterförmig angeordnet sind, wobei die Enden die Kontaktbereiche (12) bilden.

12. Vorrichtung nach Anspruch 11, wobei die Spitzen (11) mit einer geerdeten Basis (14) fest verbunden sind.

13. Vorrichtung nach Anspruch 11 oder 12, wobei die Spitzen (11) gleichmäßig beabstandet sind und nahe aneinander liegen, so dass sie ein gleichmäßiges und dichtes Gitter von Kontaktbereichen (12) auf der Aufnahmefläche (10) bilden.

14. Vorrichtung nach einem der Ansprüche 7 bis 13, wobei die Mittel (20) zum Generieren des ionisierten Gasstroms (F1) dazu geeignet sind, Impulse einstellbarer Dauer, insbesondere von 50 bis 300 ms, zu generieren.

## Claims

1. A method for analysing a device for spraying a pharmaceutical fluid product, **characterized in that** it comprises the following steps:
- providing a spray head (1) for a device for spraying a pharmaceutical fluid product, said spray head (1) comprising a spray orifice (2),
- providing a receiving surface (10) comprising a plurality of discrete contact zones (12) separated by voids (13), said contact zones (12) being electrically conductive,
- passing a flow of ionised gas (F1) through said spray orifice (2) of said spray head (1), said flow of ionised gas (F1) being charged with electrons,
- sending said flow of ionised gas (F1) onto said receiving surface (10),
- visualising the impact zone for said flow of ionised gas (F1) on said receiving surface (10), and
- analysing said visualisation of said impact zone in order to determine whether or not said impact zone complies with predetermined specifications.

2. The method as claimed in claim 1, in which said flow of ionised gas (F1) is a flow of compressed air.

3. The method as claimed in claim 1 or claim 2, in which said flow of ionised gas (F1) is a flow of ionised pulsed air.

4. The method as claimed in any one of the preceding claims, in which said step for analysis comprises determining the geometry, in particular the symmetry, of the impact zone for said flow of compressed gas (F1) on said receiving surface (10).

5. The method as claimed in any one of the preceding claims, in which said predetermined specifications comprise a predetermined planar extent of the impact zone for said flow of compressed gas (F1) on said receiving surface (10), in a manner such that the spray heads (1) for which said planar extent is similar to said predetermined planar extent are classified as compliant, and the spray heads (1) for which said planar extent is different from said predetermined planar extent are classified as non-compliant.

6. The method as claimed in any one of the preceding claims, in which an operating cycle comprises the following steps:
- connecting said receiving surface (10) to ground in order to eliminate any electrical charges,
- generating said flow of ionised gas (F1) and sending it through said spray head (1) onto said receiving surface (10),
- detecting each contact zone (12) which becomes charged with negative ions in contact with said flow of ionised gas(F1), and
- visualising said impact zone by means of a man-machine interface.

7. A device for analysing a device for spraying a pharmaceutical fluid product, **characterized in that** it comprises:
- a spray head (1) for a device for spraying a pharmaceutical fluid product, said spray head (1) comprising a spray orifice (2);
- a receiving surface (10) comprising a plurality of discrete contact zones (12) separated by voids (13), said contact zones (12) being electrically conductive,
- grounding means for eliminating any electrical charges from said receiving surface (10) before each analysis,
- means (20) for generating a flow of ionised gas (F1) in order to pass a flow of ionised gas (F1) through said spray orifice (2) of said spray head (1) and sending it onto said receiving surface (10), said flow of ionised gas (F1) being charged with electrons,
- processing means (40) for visualising the impact zone for said flow of ionised gas (F1) on said receiving surface (10), and
- means (50) for analysis for the analysis of said visualisation of said impact zone in order to determine whether or not said impact zone complies with predetermined specifications.

8. The device as claimed in claim 7, in which said flow of ionised gas (F1) is a flow of ionised pulsed air.

9. The device as claimed in claim 7 or claim 8, in which said processing means (40) comprise an amplifier and means for measuring the potential difference for each contact zone (12).

10. The device as claimed in any one of claims 7 to 9, in which each contact zone (12) of said receiving surface (10) is connected to said processing means (40) by a respective low impedance cable (30).

11. The device as claimed in any one of claims 7 to 10, in which said receiving surface (10) is formed by the ends of a plurality of points (11) disposed in an array, said ends forming said contact zones (12).

12. The device as claimed in claim 11, in which said points (11) are integral with a grounded base (14).

13. The device as claimed in claim 11 or claim 12, in which said points (11) are equidistant from and close to one another, thereby forming a regular and dense array of contact zones (12) on said receiving surface (10).

14. The device as claimed in any one of claims 7 to 13, in which said means (50) for generating a flow of ionised gas (F1) are adapted to generate pulses of adjustable duration, in particular from 50 to 300ms.
